# EUROPEAN PATENT SPECIFICATION

(11) **EP 0 621 054 B1**
(45) Date of publication and mention of the grant of the patent: **17.06.1998**
(21) Application number: 94103706.1
(22) Date of filing: 10.03.1994
(51) Int. Cl.: A61N 1/05, H01B 1/00, A61N 1/362

(54) **Electrode device**
Elektrodenvorrichtung
Dispositif d'électrodes

(30) Priority: 22.04.1993 SE 9301346
(43) Date of publication of application: 26.10.1994
(73) Proprietor: Pacesetter AB, 171 95 Solna (SE)
(72) Inventor: Högnelid, Kurt, S-137 38 Västerhaninge (SE); Neubauer, Heinz, S-175 70 Järfälla (SE); Wolf, Jens, S-121 46 Johanneshov (SE)
(74) Representative: Lettström, Richard Wilhelm

(56) References cited:
- DE-A- 3 640 033
- US-A- 3 412 198
- US-A- 3 601 519
- US-A- 3 884 243
- US-A- 5 099 855

## Description

This invention relates to an electrode device comprising an insulating sleeve in which an electrical conductor is arranged to electrically connect a contact at a proximal part of the electrode device to an electrode surface on the electrode device.

Electrode devices are used when e.g. a pacemaker or a defibrillator is to be connected to a heart to sense the heart's function or treat the heart. As a rule, these electrode devices are composed of one or more helically wound metal wires running from a contact at a proximal end, connectable to the pacemaker or defibrillator, to an electrode surface connected to or near the heart. Such an electrode device is, for example, disclosed in DE-A-3 640 033. The electrode surface can e.g. consist of a small pace electrode, a larger intracardiac defibrillation electrode or an epicardiac patch electrode. The electrode devices can also be equipped with different kinds of sensors which are connected, via a metal conductor, to the pacemaker or defibrillator.

Since the heart is in constant motion, electrode devices connected to or near the heart are constantly exposed to changes in load. So one or more of the metal wires could fracture, thereby breaking the electrical connection between an electrode surface or a sensor and the contact at the proximal end. When a plurality of metal wires, electrically insulated from one another, are used, the insulation could sustain abrasion damage caused by the constantly shifting load, causing a short-circuit. In addition, fabrication of the electrode device becomes more complex as the number of incorporated components increases, as when a plurality of electrode surfaces and sensors have to be connected to one and the same electrode device.

One object of the invention is to achieve an electrode device, according to the introduction above, which is capable of reliably and safely connecting the contact and electrode surfaces electrically, or interconnecting sensors, without the risk of fractures, short-circuits or the like and which is implantable.

Another object of the invention is to achieve an electrode device for which the number of components can be reduced without any reduction in the number of functions the electrode device can perform.

These objects are achieved with an implantable electrode device in accordance with the invention in that the electrical conductor is fluid.

A fluid conductor is a completely reliable carrier of energy and signals between the contact and electrode surface or a sensor. A fluid conductor can withstand an infinite number of load changes without any break occurring, and a thin layer of insulation is sufficient to prevent short-circuits, since a fluid conductor would impose lighter abrasion loads on the insulation than a solid conductor.

The fluid conductor can advantageously be non-metallic.

A refinement of the electrode device is achieved in accordance with the invention in that the insulating sleeve is devised with a through hole between the contact and the electrode surface, and the through hole is filled with an electrically conductive gel to form the electrical conductor.

Fabrication of the electrode device is simpler, because the through hole in the sleeve only needs to be filled with the fluid gel.

When the electrode device is for defibrillation systems devised as patches attached to or applied near the heart, it is advantageous if the distal end comprises a first conductive elastic plate and a second elastic plate joined at the edges, thereby forming a cavity between the plates, said cavity being filled with an electrically conductive gel, and the conductive electrical plate and the electrically conductive gel are electrically connected to the electrical conductor, the conductive plate then forming the electrode surface.

This produces a patch electrode which is supple and compliant. When the electrode surface is e.g. applied directly to the heart, the gel will smoothly comply with heart movements, in contrast to patches devised with e.g. metal wires.

In conjunction with the use of an electrically conductive gel, it is advantageous if the electrically conductive gel consists of a doped polymer gel or a hydrophilic gel. The gel can particularly be doped with a metallic powder, salt or powdered carbon

As an alternative to the use of an electrically conductive gel, the electrode device can be devised in accordance with the invention in such a way that the insulating sleeve has a through hole between the contact and the electrode surface, and the through hole is filled with an electrolyte. The use of an electrolyte would be especially appropriate for use in sensing functions.

In conjunction herewith, it is advantageous if the through hole is filled at fabrication with undissolved ions.

When the device is implanted in the body, the through hole will fill with body fluid which diffuses in through the sleeve. The undissolved ions deposited in the hole prior to the implantation will then form a solution with the fluid, thereby achieving an electrolyte in the hole. This process occurs within a short time after implantation and is generally completed within a few days.

Alternately, the electrode surface can be made of an ion-transporting material.

If the electrode surface is made from an ion-transporting material, no undissolved ions will be needed during fabrication. The electrolyte is formed in the through hole by the migration of ions through the ion-transporting material.

A refinement of the electrode device is achieved in accordance with the invention in that the electrode device further comprises at least one additional electrical conductor to connect at least one additional contact at the proximal end to at least one additional electrode surface.

In principle, the electrode device can encompass an unlimited number of conductors. They can even be a mixture of solid, metal conductors and fluid conductors. When e.g. large energies have to be transferred, as in emission of defibrillation pulses, an arrangement of solid, metal conductors, or a combination of solid, metal conductors and fluid conductors might be appropriate. The electrode device could also be devised so it only contains fluid conductors.

In view of the above, it is also possible in accordance with the invention to devise the electrode device so it comprises an insulating sleeve with an electrical conductor to electrically connect a contact at a proximal end of the electrode device with an electrode surface on the electrode device, wherein the electrode device at the distal end comprises a first conductive elastic plate and a second elastic plate joined at the edges, thereby forming a cavity between the plates, said cavity being filled with an electrically conductive gel, and the conductive elastic plate and the electrically conductive gel are electrically connected to the electrical conductor, the conductive plate then forming the electrode surface.

The invention will now be described in greater detail, referring to 7 figures in which
FIG. 1 shows a first embodiment of the electrode device according to the invention;
FIG. 2 shows details of the way the electrode device, according to the first embodiment, could be constructed;
FIG. 3 shows an alternative version of the electrode device according to the first embodiment;
FIG. 4 shows a second embodiment of the electrode device according to the invention;
FIG. 5 shows details of the way the electrode device, according to the second embodiment, could be constructed;
FIG. 6 shows a third embodiment of the electrode device according to the invention;
FIG. 7 shows an embodiment of a defibrillation electrode according to the invention.

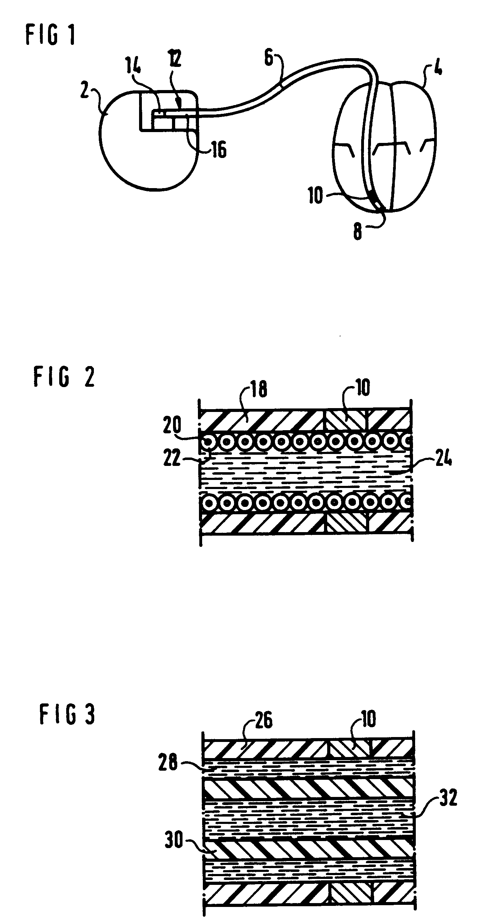

In FIG. 1 is shown a pacemaker 2 connected to a heart 4 by an electrode device 6. The electrode device 6 is a VVI device, i.e. it has a tip electrode 8 placed in the right ventricle to stimulate the heart 4 and a ring electrode 10 to sense heart signals in the ventricle. The electrode device 6 also has a contact appliance 12 for detachable connection to the pacemaker 2. The contact appliance 12 has a first contact surface 14 connected to the tip electrode 8 and pace electronics and a second contact surface 16 connected to the ring electrode 10 and pace electronics.

In FIG. 2 is shown a section of the electrode device 6. A metal conductor 20 is helically arranged in an insulating sleeve 18. The metal conductor 20 has a coating of insulation 22 to insulate the metal conductor from the electrolyte 24 which fills the through hole in the insulating sleeve 18. The ring electrode 10 is an integral part of the insulating sleeve 18. The ring electrode 10 can be e.g. a platinum electrode, or a membrane made of a conductive material, in contact with the electrolyte 24 and surrounding body tissue in order to receive and transmit electrical signals to the pacemaker.

In FIG. 3 is shown an alternative version of the electrode device 6. A first insulating sleeve 26 encloses the entire electrode device 6. An insulating tube 30 is located inside this sleeve 26. A first fluid conductor 28 is located in the space between the tube 30 and the sleeve 26. The fluid conductor 28 can consist of an electrolyte or a conductive gel. A second fluid conductor 32 is located in the insulating tube 30 for connection of the tip electrode 8 to the pacemaker 2. The second fluid conductor 32 consists of a conductive gel, e.g. a doped polymer gel. Even here, the ring conductor 10 can be a metal ring or a membrane made from some conductive material.

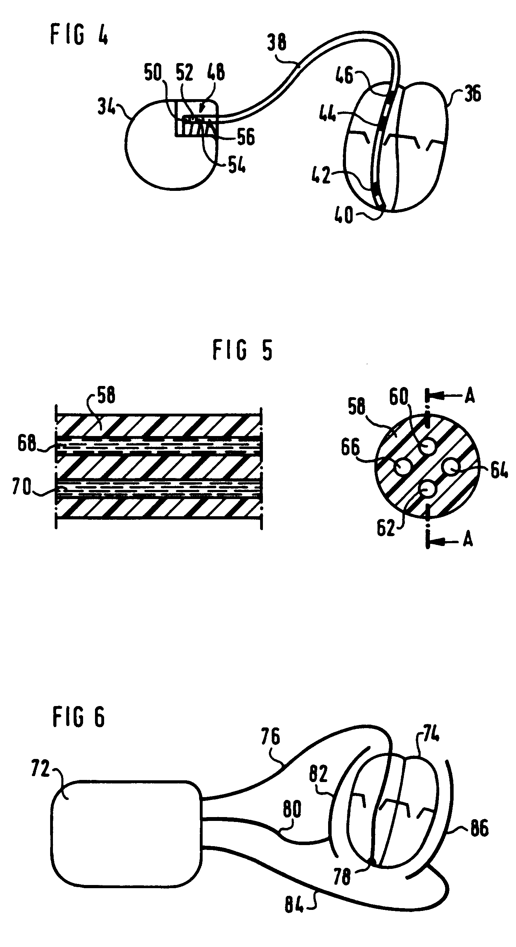

In FIG. 4 is shown a second embodiment of the electrode device according to the invention. A pacemaker 34 is connected to a heart 36 by an electrode device 38. The electrode device 38 is devised as a VDD electrode, i.e. it comprises a tip electrode 40 placed in the right ventricle to stimulate heart tissue, a first ring electrode 42 for sensing heart signals in the ventricle and a second ring electrode 44 and a third ring electrode 46 for sensing atrial activity in the heart. The electrode device 38 is detachably connected to the pacemaker by a contact appliance 48. The contact appliance 48 has a first contact surface 50 which is connected to the tip electrode 40, a second contact surface 52 connected to the first ring electrode 42, a third contact surface 54 connected to the second ring electrode 44 and a fourth contact surface 56 connected to the third ring electrode 46.

In FIG. 5 is shown, to the right, a cross-section of the electrode device 38 and, to the left, a part of a longitudinal cross-section of the electrode device 38. The electrode device 38 comprises an insulating sleeve 58 in which four through holes 60, 62, 64, 66 are arranged. Each hole 60, 62, 64, 66 connects the respective tip electrode 40, the first ring electrode 42, the second ring electrode 44 and the third ring electrode 46 (FIG. 4) to the pacemaker 34. The first cavity 60, connecting the tip electrode 40 to the first contact surface 50, is filled with an electrically conductive gel 68. The other cavities 62, 64, 66 can be filled with a conductive gel or an electrolyte.

In FIG. 6 is shown a defibrillator 72 connected to a heart via a first electrode device 76 which has a tip electrode 78 connected to the right ventricle of the heart 74. The defibrillator 72 can, via the first electrode device 76, supply the heart with the same treatment as a pacemaker. The first electrode device 76 only has one electrical conductor from the defibrillator 72 to the tip electrode 78. This electrical conductor can be devised with an electrically conductive gel in any of the previously described ways. In order to defibrillate the heart 74, the defibrillator 72 comprises a second electrode device 80 and a third electrode device 84. The second electrode device 80 has a first defibrillation electrode 82 designed to be placed directly on or near the heart 74. The third electrode device 84 has a second defibrillation electrode 86 which, in the corresponding way as the first defibrillation electrode 82, is placed directly on or near the heart 74. A defibrillation pulse from the defibrillator 72 is delivered across the first defibrillation electrode 82 and the second defibrillation electrode 86 so the pulse passes through the heart 74. The second electrode device 80 and the third electrode device 84 can have metallic or non-metallic fluid conductors.

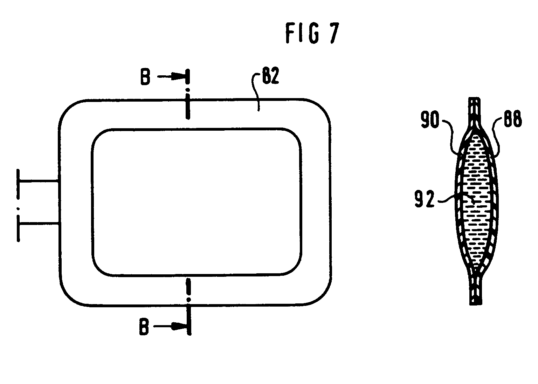

In FIG. 7 is shown the first defibrillation electrode 82 in greater detail. The first defibrillation electrode 82 has a first electrode plate 88, made from a conductive material, and a second electrode plate 90, which can be conductive or insulating. The two plates 88, 90 are joined at the edges, thereby forming a cavity which is filled with an electrically conductive gel 92. The first defibrillation electrode 82 is applied to the heart 74 with the conductive plate 88 on the heart 74. The gel-filled space makes the first defibrillation electrode 82 supple and adaptable to the movements of the heart 74.

## Claims

1. An implantable electrode device (6; 38; 76) comprising a contact (12; 48) at a proximal part of the electrode device (6; 38; 76), an electrode surface (8, 10; 40, 42, 44, 46; 78), an insulating sleeve (18; 26; 58) in which an electrical conductor (24; 28; 68, 70) is arranged to electrically connect, said contact (12, 48) to the electrode surface (8, 10, 40, 42, 44, 46, 78) characterized in that the electrical conductor (24; 28, 32; 68, 70) is fluid.

2. An electrode device according to claim 1, characterized in that the fluid conductor (24; 28, 32; 68, 70) is non-metallic.

3. An electrode device according to claim 1 or 2, characterized in that the insulating sleeve (18; 26; 58) is devised with a through hole between the contact (12; 48) and the electrode surface (8, 10; 40, 42, 44, 46; 78), and the through hole is filled with an electrically conductive gel to form the electrical conductor.

4. An electrode device according to any of claims 1 - 3, characterized in that a distal end of the electrode device has a conductive elastic plate (88) and an elastic plate (90) joined at the edges, thereby forming a cavity between the plates (88, 90), said cavity being filled with an electrically conductive gel (92), and the conductive elastic plate (88) and the electrically conductive gel (92) are electrically connected to the electrical conductor, the conductive plate then forming the electrode surface (8, 10, 40, 42, 44, 46, 78).

5. An electrode device according to claim 3 or 4, characterized in that the electrically conductive gel consists of a doped polymer gel or a hydrophilic gel.

6. An electrode device according to claim 5, characterized in that the gel is doped with a metallic powder, salt or powdered carbon.

7. An electrode device according to claim 1 or 2, characterized in that the insulating sleeve (18, 26, 58) is devised with a through hole (60, 62, 64, 66) between the contact (12, 48) and the electrode surface (8, 10, 40, 42, 44, 46, 78), and the through hole is filled with an electrolyte.

8. An electrode device according to claim 7, characterized in that the through hole (60, 62, 64, 66) is filled at fabrication with undissolved ions.

9. An electrode device according to claim 7, caracterized in that the electrode surface (8, 10, 40, 42, 44, 46, 78) is made of an ion-transporting material.

10. An electrode device according to any of the above claims, characterized in that there is at least one additional electrical conductor (32, 68, 70) to connect at least one additional contact at the proximal end to at least one additional electrode surface (8, 10, 40, 42, 44, 46, 78).

11. An electrode device according to claim 10, characterized in that the additional conductor (32, 68, 70) is fluid.

12. An electrode device according to claim 1 characterized in that said conductor (24, 28, 32, 68, 70) is an electrically conductive gel and that the electrode device at a distal end comprises a first conductive, elastic plate (88) and a second elastic plate (90) joined at the edges, thereby forming a cavity between the plates (88, 90), said cavity being filled with an electrically conductive gel (92), and the conductive elastic plate (88) and the electrically conductive gel (92) are electrically connected to the electrical conductor (24, 28, 32, 68, 70), the conductive plate (88) then forming the electrode surface (82).

## Patentansprüche

1. Eine implantierbare Elektrodenvorrichtung (6; 38; 76) mit einem Kontakt (12; 48) an einem proximalen Teil der Elektrodenvorrichtung (6; 38; 76), einer Elektrodenoberfläche (8, 10; 40, 42, 44, 46; 78), einer isolierenden Hülle (18; 26; 58), in der ein elektrischer Leiter (24; 28; 68, 70) angeordnet ist, um den Kontakt (12; 48) mit der Elektrodenoberfläche (8, 10; 40, 42, 44, 46; 78) elektrisch zu verbinden, **dadurch gekennzeichnet,** daß der elektrische Leiter (24; 28, 32; 68, 70) flüssig ist.

2. Eine Elektrodenvorrichtung nach Anspruch 1, **dadurch gekennzeichnet,** daß der flüssige Leiter (24; 28, 32; 68, 70) nichtmetallisch ist.

3. Eine Elektrodenvorrichtung nach Anspruch 1 oder 2, **dadurch gekennzeichnet,** daß die isolierenden Hülle (18; 26; 58) mit einem Durchgangsloch zwischen dem Kontakt (12; 48) und der Elektrodenoberfläche (8, 10; 40, 42, 44, 46; 78) versehen ist und das Durchgangsloch mit einem elektrisch leitenden Gel gefüllt ist, um den elektrischen Leiter zu bilden.

4. Eine Elektrodenvorrichtung nach einem der Ansprüche 1 - 3, **dadurch gekennzeichnet,** daß ein distales Ende der Elektrodenvorrichtung eine leitende elastische Platte (88) und eine elastische Platte (90) aufweist, die an den Kanten verbunden sind, wodurch sie einen Hohlraum zwischen den Platten (88, 90) bilden, der mit einem elektrisch leitenden Gel (92) gefüllt ist, und die leitende elastische Platte (88) und das elektrisch leitende Gel (92) elektrisch mit dem elektrischen Leiter verbunden sind, wodurch die leitende Platte dann die Elektrodenoberfläche (8, 10; 40, 42, 44, 46; 78) bildet.

5. Eine Elektrodenvorrichtung nach Anspruch 3 oder 4, **dadurch gekennzeichnet,** daß das elektrisch leitende Gel aus einem dotierten polymeren Gel oder einem hydrophilen Gel besteht.

6. Eine Elektrodenvorrichtung nach Anspruch 5, **dadurch gekennzeichnet,** daß das Gel mit einem metallischen Pulver, Salz oder Kohlenstoffpulver dotiert ist.

7. Eine Elektrodenvorrichtung nach Anspruch 1 oder 2, **dadurch gekennzeichnet,** daß die isolierende Hülle (18, 26, 58) mit einem Durchgangsloch (60, 62, 64, 66) zwischen dem Kontakt (12; 48) und der Elektrodenoberfläche (8, 10; 40, 42, 44, 46; 78) versehen ist und das Durchgangsloch mit einem Elektrolyten gefüllt ist.

8. Eine Elektrodenvorrichtung nach Anspruch 7, **dadurch gekennzeichnet,** daß das Durchgangsloch (60, 62, 64, 66) bei der Herstellung mit ungelösten Ionen gefüllt wird.

9. Eine Elektrodenvorrichtung nach Anspruch 7, **dadurch gekennzeichnet,** daß die Elektrodenoberfläche (8, 10; 40, 42, 44, 46; 78) aus einem Ionen transportierenden Material besteht.

10. Eine Elektrodenvorrichtung nach einem der vorhergehenden Ansprüche, **dadurch gekennzeichnet,** daß es mindestens einen zusätzlichen elektrischen Leiter (32, 68, 70) gibt, um mindestens einem zusätzlichen Kontakt an dem proximalen Ende mit mindestens einer zusätzlichen Elektrodenoberfläche (8, 10; 40, 42, 44, 46; 78) zu verbinden.

11. Eine Elektrodenvorrichtung nach Anspruch 10, **dadurch gekennzeichnet,** daß der zusätzliche Leiter (32, 68, 70) flüssig ist.

12. Eine Elektrodenvorrichtung nach Anspruch 1, **dadurch gekennzeichnet,** daß der Leiter (24, 28, 32, 68, 70) ein elektrisch leitendes Gel ist und daß die Elektrodenvorrichtung (6; 38; 76) an einem distalen Ende eine erste leitende, elastische Platte (88) und eine zweite elastische Platte (90) aufweist, die an den Kanten verbunden sind, wodurch sie einen Hohlraum zwischen den Platten (88, 90) bilden, der mit einem elektrisch leitenden Gel (92) gefüllt ist, und die leitende elastische Platte (88) und das elektrisch leitende Gel (92) elektrisch mit dem elektrischen Leiter (24, 28, 32, 68, 70) verbunden sind, wodurch die leitende Platte (88) dann die Elektrodenoberfläche (82) bildet.

## Revendications

1. Dispositif (6;38;76) implantable à électrode comprenant un contact (12,48) en une partie proximale du dispositif (6;38;76) à électrode, une surface (8,10;40,42,44,46;78) d'électrode, un manchon (18,26,58) isolant dans lequel est placé un conducteur (24;28;68,70) électrique pour relier électriquement le contact (12,48) à la surface (8,10;40,42,44,46;78) d'électrode, caractérisé en ce que le conducteur (24;28,32;68,70) électrique est du fluide.

2. Dispositif à électrode suivant la revendication 1, caractérisé en ce que le conducteur (24;28,32;68,70) sous forme de fluide n'est pas métallique.

3. Dispositif à électrode suivant la revendication 1 ou 2 caractérisé en ce que le manchon (18;26;58) isolant est muni d'un trou traversant entre le contact (12;48) et la surface (8,10;40,42,44,46;78) d'électrode et le trou traversant est empli d'un gel électriquement conducteur pour former le conducteur électrique.

4. Dispositif à électrode suivant l'une quelconque des revendications 1 à 3, caractérisé en ce qu'une extrémité distale du dispositif à électrode a une plaque (88) conductrice élastique et une plaque (90) élastique réunies sur les bords en formant une cavité entre les plaques (88,90), la cavité étant emplie d'un gel (92) conducteur de l'électricité et la plaque (88) conductrice élastique et le gel (92) conducteur de l'électricité sont reliés électriquement au conducteur électrique, la plaque conductrice formant ainsi alors la surface (8,10,40,42,44,46,78) d'électrode.

5. Dispositif à électrode suivant la revendication 3 ou 4, caractérisé en ce que le gel conducteur d'électricité consiste en un gel polymère dopé ou en un gel hydrophile.

6. Dispositif à électrode suivant la revendication 5, caractérisé en ce que le gel est dopé par une poudre métallique, un sel ou du carbone pulvérulent.

7. Dispositif à électrode suivant la revendication 1 ou 2, caractérisé en ce que le manchon (18,26,38) isolant est muni d'un trou (60,62,64,66) traversant entre le contact (12,48) et la surface (8,10,40,42,44,46,78) d'électrode et le trou traversant est empli d'un électrolyte.

8. Dispositif à électrode suivant la revendication 7, caractérisé en ce que le trou (60,62,64,66) traversant est empli à la fabrication dons non dissous.

9. Dispositif à électrode suivant la revendication 7, caractérisé en ce que la surface (8,10,40,42,44,46,78) d'électrode est en une matière transportant des ions.

10. Dispositif à électrode suivant l'une quelconque des revendications précédentes caractérisé en ce qu'il y a au moins un conducteur (32,68,70) électrique supplémentaire destiné à relier au moins un contact supplémentaire à l'extrémité proximale à au moins une surface (8,10,40,42,44,46,48) d'électrode supplémentaire.

11. Dispositif à électrode suivant la revendication 10, caractérisé en ce que le conducteur (32, 68,70) supplémentaire est du fluide.

12. Dispositif à électrode suivant la revendication 1, caractérisé en ce que le conducteur (24;28;32;68;72) est un gel conducteur d'électricité et en ce que le dispositif (3) à électrode comprend à une extrémité distale une plaque (88) élastique conductrice et une plaque élastique réunies sur leurs bords en formant une cavité entre les plaques (88,90), la cavité étant emplie d'un gel (92) conducteur de l'électricité et la plaque (88) élastique conductrice et le gel (92) conducteur de l'électricité sont reliés électriquement au conducteur (24,28,32,68,70) électrique, la plaque (88) conductrice formant ainsi la surface (82) d'électrode.
